# EUROPEAN PATENT APPLICATION

(11) **EP 1 106 200 A2**
(43) Date of publication of application: **13.06.2001**
(21) Application number: 00310592.1
(22) Date of filing: 29.11.2000
(51) Int. Cl.: A61M 25/00

(54) **Manufacturing jig for medical treatment tube**

(30) Priority: 30.11.1999 JP 34108299
(71) Applicant: Piolax Medical Devices, Inc., Yokohama-shi, Kanagawa-ken (JP)
(72) Inventor: Sakamoto, Katsumi, Hodogaya-ku, Yokohama-shi, Kanagawa-ken (JP); Ohkata, Ichizou, Hodogaya-ku, Yokohama-shi, Kanagawa-ken (JP); Takahashi, Hiroshi, Hodogaya-ku, Yokohama-shi, Kanagawa-ken (JP)
(74) Representative: Abbie, Andrew Kenneth

(57) **Abstract**

A recessed part is configured to insert the tube to be held. A guide part is configured to bring a cutter in contact with a side face of the tube to be held in the recessed part.

## Description

### BACKGROUND OF THE INVENTION

### 1. Field of the Invention

The present invention relates to a manufacturing jig for a medical treatment tube which is formed with a hole or a slit to flow out a liquid medicine and so on at a predetermined place of a circumferential wall of the medical treatment tube, such as a catheter.

### 2. Description of Relevant Art

When dosing a medical liquid or taking out a body fluid through a catheter inserted in a body, there occurs forming a hole or a slit at a predetermined place of a circumferential wall of a catheter.

There has been disclosed in Japanese Patent Application Laid-Open Publication No. 10-2721187 a catheter to be fixed and placed in which a catheter body has inside a distal end thereof a wire which is bent greater than an outer size of the catheter body, having a slit-shaped opening at a circumferential wall on a near side at a distance from the distal end.

The catheter is inserted in a blood vessel through a main catheter which has been previously inserted in it, being pushed out of the main catheter, and its distal end is pressed to an inner wall of the main catheter to be fixed by an elastic restoring force of the wire. By pulling out the main catheter at its outer circumference, the catheter is fixed and placed in the blood vessel. At this time. a slit-shaped opening makes a fixed and placed position adjusted to locate at a branch part of the blood vessel to introduce to, for example, a cancer tissue, so that when dosing a carcinostatic agent, the carcinostatic agent can effectively flows to the cancer tissue.

In this case, a distance from the distal end of the catheter to an opening for flowing out a liquid medicine need to change in dependent on a patient or a patient position, and a doctor works to form a hole at a desired position by scissors before or during operation every time. However, a surface of the catheter is coated with a hydrophilic resin to be slippery. and it was remarkably difficult to form a hole of a proper size at a desired place.

On the other hand, for example, Japanese Patent Application Laid-Open Publication No. 7-213616, NO. 7-214498, 7-205098, and 6-106625 disclose a method and a device in which a hole is formed to a circumferential wall of a tube, such as a catheter, by means of an ultrasonic welder, high-frequency welder, or a stamping-out blade with a heating device.

### SUMMARY OF THE INVENTION

However, the conventional method or device for forming a hole needs an ultrasonic oscillator, a high frequency heating device, or a heater, and the device becomes large-scale, so that it does not suite for use for a doctor to easily form a hole before or during an operation.

An object of the present invention is to provide a manufacturing jig for a medical treatment tube which forms a hole or a slit of a size formed at a desired place on a circumferential wall of a tube for a medical treatment such as a catheter easily and reliably.

To achieve the object, a first aspect of the invention provides a manufacturing jig for a medical treatment tube which includes a recessed part which is configured to insert the tube to be held, and a guide part which is configured to bring a cutter in contact with a side face of the tube to be held in the recessed part.

According to the first aspect. the tube is inserted in the recessed part for holding, and in the sate the cutter is adapted to the guide part. By contacting a distal end of the blade with a circumferential wall of the tube, at a desired place on the circumferential wall of the tube, a hole or a slit of a size is formed easily and reliably.

Preferably, the cutter has a cylindrical or an arc shaped blade. The guide part has a cylindrical or an arc shaped inner wall for guiding the blade. When the cutter is inserted in the guide part, the guide part is formed such that a part of the blade contacts in an arc shape with a part of a circumferential wall of the tube in a crossing direction to a longitudinal direction of the tube.

According to the aspect, a cutter which has a cylindrical blade such as a trepan for biopsy is inserted in the inner wall of the guide part, and a part of the circumferential wall of the tube is cut in an arc shape in a crossing direction to the longitudinal direction of the tube, thereby being formed with a hole of a certain size in a shape similar to a circle or an ellipse.

Preferably, the cutter has a blade in a straight line. The guide part is formed in a straight line along a longitudinal direction of the tube.

According to the aspect, a cutter in a flat plate such as a scalpel is adapted to and slid on the guide part, and along the longitudinal direction of the tube, a slit in a straight line is formed.

Preferably, the recessed part has a straight line shaped part for straightly holding the tube. The guide part is provided to the straight line shaped part.

According to the aspect, when the tube is held in the recessed part, the guide part is provided to straightly a held part, which is to be cut. Therefore, a shape of the tube is kept, and a shape or a size of the cut hole or slit is further easy to be fixed.

Preferably, the recessed part has a curved part with a curvature for bending and holding the tube without buckling. The curved part has the guide part provided on an inner or an outer circumference thereof.

According to the aspect, when the curved part of the tube bent and held is cut on the inner circumference, an elongated hole is formed to the tube in a longitudinal direction of the tube. On the other hand, when it is cut on the outer circumference, a shortened hole is formed to the tube in the longitudinal direction.

Preferably, the recessed part has a width smaller than a diameter of the tube and capable of inserting the tube under pressure, and has a depth for a part of a circumferential wall of the tube to project from an opening edge thereof when inserting the tube under pressure.

According to the aspect, the tube is inserted in the recessed part under pressure, being securely held without generation of rotation or shift at the time of cutting. In addition, when inserting the tube in the recessed part, a part of a circumferential wall projects from the opening edge of the recessed part. Thus, the tube is securely inserted, with it being pressed.by fingers.

Preferably, width narrowed parts are provided at opposite positions relative to a part of the recessed part provided with a guide part, for the tube to be held further tightly.

According to the aspect, at both sides of the guide part corresponding to a part for cutting the tube, due to holding the tube further tightly, the parts in which a width of the recessed part is narrowed are provided. Therefore, the tube is effectively prevented from shift and rotation when the cutter is brought in contact the tube, and a hole or a slit in a fixed shape is formed.

Preferably, the recessed part has a wall, and the guide part opens the wall.

Preferably, the wall straightly extends.

Preferably, the wall is curved.

Preferably, the guide part extends along the wall.

Preferably, the guide part opens an end of the wall.

Preferably, the recessed part is narrowed at a place thereof.

As for the aspect. the guide part may have a triangular, rectangular shape, a polygonal shape, a curved shape, an arc shape, or a circular shape in section. The recessed part may have a triangular shape, a rectangular shape, a polygonal shape, a curved shape, or an arc shape in transverse section and may have a straight line, a curved shape, or a zigzag line in a longitudinal direction thereof.

Regarding the invention, if a straight line shaped part of the recessed part is provided with a guide part, at opposite positions relative to the guide part in the longitudinal direction, the recessed part is preferably provided with zigzagged parts, so that holding forces for the tube on both side parts of the guide part are further enhanced.

In addition, if the guide part is in a straight line along the longitudinal direction of the tube, it is preferable that a core member is inserted in an inner part of the tube and in the state the cutter is adapted to the guide part to form a slit, so that opposite wall parts of the tube are prevented from being cut.

### BRIEF DESCRIPTION OF THE ACCOMPANYING DRAWINGS

The above and further objects and novel features of the present invention will more fully appear from the following detailed description when the same is read in conjunction with the accompanying drawings, in which:
Fig. 1 is a perspective view showing a manufacturing jig for a medical treatment tube with a cylindrical cutter of an embodiment according to the invention;
Fig. 2 is a perspective view showing a manufacturing jig for a medical treatment tube with a flat-plate shaped cutter, as observed in a direction different from Fig. 1:
Figs. 3A and 3B are cross-sectional views of a manufacturing jig for a medical treatment tube, Fig. 3A is a cross-sectional view taken along A1-A1 line of Fig. 1, and Fig. 3B is a cross-sectional view taken along B1-B1 line of Fig. 1;
Fig. 4 is a cross-sectional view showing a state where a manufacturing jig for a medical treatment tube holds a medical treatment tube;
Figs. 5A and 5B show a hole and a slit formed to a medical treatment tube by a manufacturing jig for a medical treatment tube, Fig. 5A is a side view showing a hole shape formed by the manufacturing jig, and Fig. 5B is a side view showing a slit shape formed by the manufacturing jig;
Figs. 6A and 6B are perspective views of a manufacturing jig for a medical treatment tube according to another embodiment of the invention, as observed in respective different directions;
Fig. 7 is an explanation view showing hole shapes when a medical treatment tube is cut at an outer circumferential side and an inner circumferential side of a bent portion thereof;
Fig. 8 is a plan view showing a manufacturing jig for a medical treatment tube of further another embodiment according to the invention; and
Fig. 9 is a plan view showing a manufacturing jig for a medical treatment tube of furthermore another embodiment according to the invention.

### DETAILED DESCRIPTION OF THE PREFERRED EMBODIMENTS

There will be detailed below the preferred embodiments of the present invention with reference to the accompanying drawings.

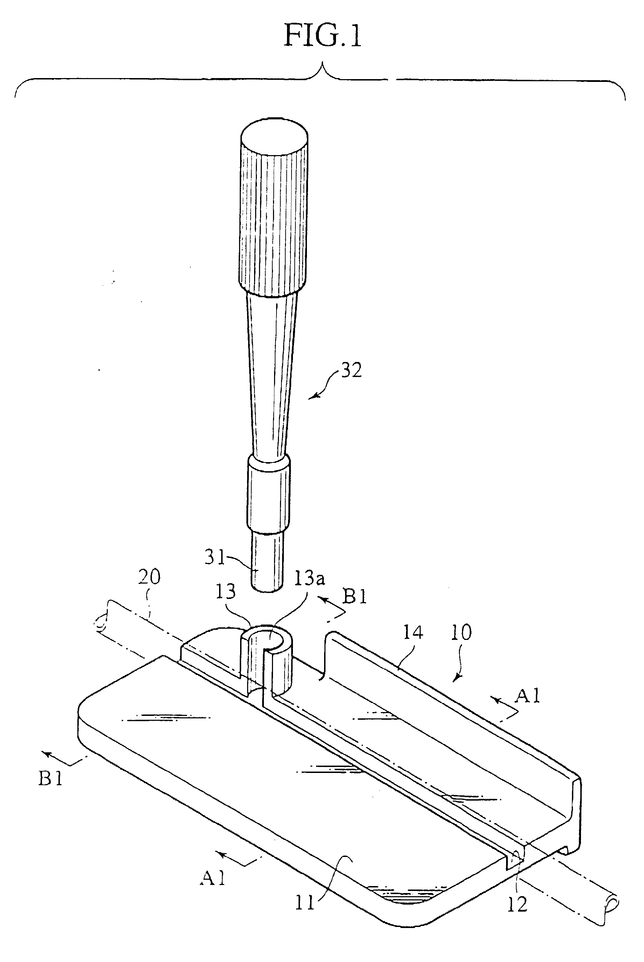

Figs. 1 to 5 show a manufacturing jig for a medical treatment tube of an embodiment according to the invention.

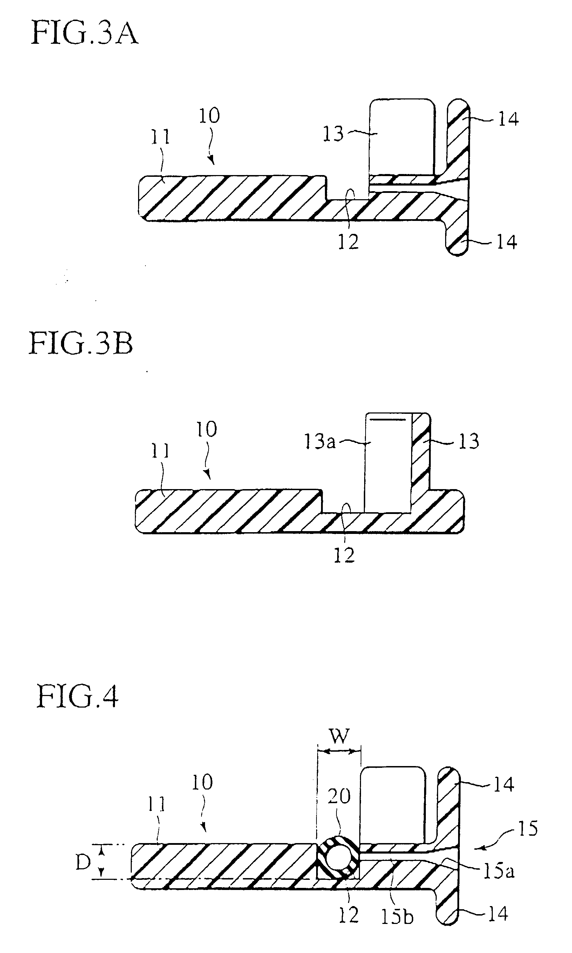

The manufacturing jig 10 for a medical treatment tube, as shown on Fig. 1, is integrally formed of a resin, having base 11 in a rectangular plate shape. The base 11 has a recessed part 12 is formed at a position a little close to a side relative to a central part in a transverse direction thereof, the recessed part extending in a straight line in a longitudinal direction. The recessed part 12, as shown on Fig. 4, has slightly a smaller width W and slightly a shallower depth D than a diameter of the medical treatment tube 20. When the medical treatment tube 20 is pushed in the recessed part 12, the tube 20 is elastically deformed to be pressingly fitted in it and a part of its circumferential wall projects from a surface on the base 11 to be easily pressed by fingers.

The recessed part 12 has a cylindrical guide part 13 in an arc sectional shape extending normally to the base 11 formed at a part of the route thereof. The guide part 13 has a shape such that a part of a cylinder is cut out along an edge of the recessed part 12. A part of a circle adapted to an inner circumference 13a is positioned to enter into the recessed part 12.

In the guide part 13, a cutter 32 with a cylindrical blade 31 is inserted so as to cut a part of a circumferential wall of the tube 20 in an arc shape in a lateral direction. A trepan for biopsy which is the existing tool may be adapted for the cutter 32. The guide part 13 has an inner size which is set for adapting the blade 31 of the cutter 32 to be inserted.

The guide 13 may have perfectly a cylindrical shape in at least an upper half part if the lower edge part thereof has an opening for inserting the tube 20 in the recessed part 12. The blade 31 of the cutter 32 is not limited to a cylindrical shape but may have an arc shape like a chisel. In that case, a R-shaped part of an arc is to be entered in the recessed part 12.

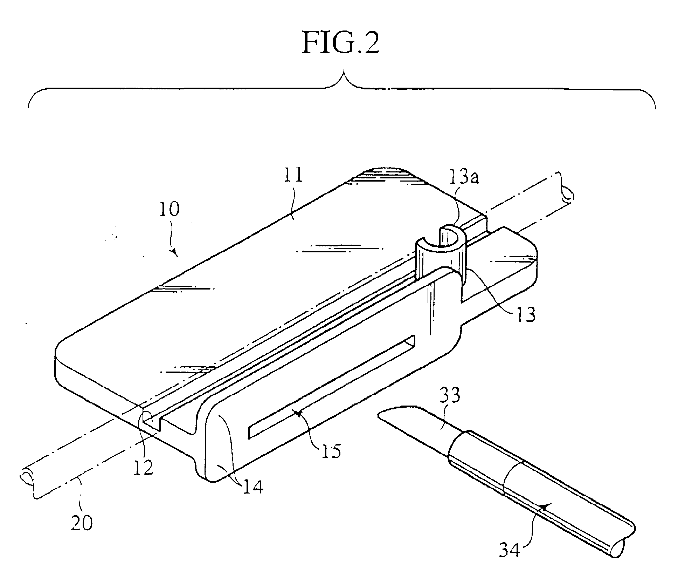

The base 11 has vertically a projecting rib 14 formed at an edge thereof parallel to the recessed part 12. The rib 14 has a side face including a part positioned within a thickness of the base 11, the part, as specially shown on Figs. 2 and 4, being formed with a straight line shaped guide part 15 defining a slit in a straight line opening to an inner face of the recessed part 12. The guide part 15 is formed such that the cutter 34 with a blade 33 in a flat plate is inserted. For this cutter 34, a scalpel is adapted.

The guide part 15 has an insertion opening part 15a formed in a tapered shape so as to be gradually widened toward an entrance side and an inner part 15b with a predetermined width to be adapted to the blade 33 of the cutter 34. The inner part 15b has a distal end opening to an inner wall of the recessed part 12.

Next, a using method of the manufacturing jig 10 for a medical treatment tube is explained. In use, in addition to the manufacturing jig 10, the cutter 32 as shown on Fig. 1 and/or the cutter 34 as shown on Fig. 2 are prepared.

For the tube 20, a catheter to be fixed and placed as shown by Japanese Patent Application Laid-Open Publication No. 10-2721187 is preferably adapted. In addition, another catheter which need to be formed with a slit or a hole on a circumferential wall. a drainer tube, or a transport line can be adapted.

At first, the tube 20 to be manufactured for making a hole is inserted and held in the recessed part 12 of the manufacturing jig 10. At this time, if a hole is formed to the tube 20, the spot is positioned to the cylindrical guide part 13, and if a slit is formed to the tube 20, the spot is positioned to the straight line shaped guide Part 15. When the tube 20 is inserted in the recessed part 12, with being pressed by fingers, as described above. a width of the recessed part 12 is narrowed than the diameter of the tube 20, and the tube 20 is inserted in the recessed part 12 under pressure, thus being securely held.

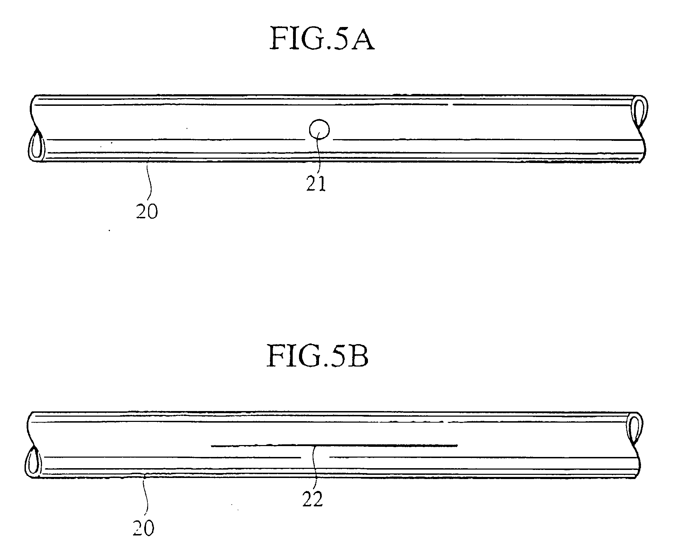

Next, if a hole is formed to the circumferential wall of the tube 20, inserting and holding the tube 20 in the recessed part 12, and with being slidably in contact with the inner wall of the cylindrical guide part 13, the cylindrical blade 31 of the cutter 32 is inserted, thereby cutting one side of the circumferential wall of the tube 20 in an arc shape in a plane view. Thus, the cut tube 20, as shown on Fig. 5A, is formed with a circular hole 21.

On the other hand, if a slit is formed to the circumferential wall of the tube 20, at first, a core member such as a guide wire as not shown on Figs is inserted in the tube 20. In the state, the tube 20 is inserted and held in the recessed part 12, the blade 33 in a flat plate is inserted through the guide part 15, and a cut is formed along a longitudinal direction of the tube 20. Although the core member is not always necessary, it is preferably inserted due to preventing the blade 33 from getting to an opposite wall part of the circumferential wall of the tube 20. Thus, as the cut is formed, the tube 20, as shown on Fig. SB, has a straight line shaped slit 22 formed thereto.

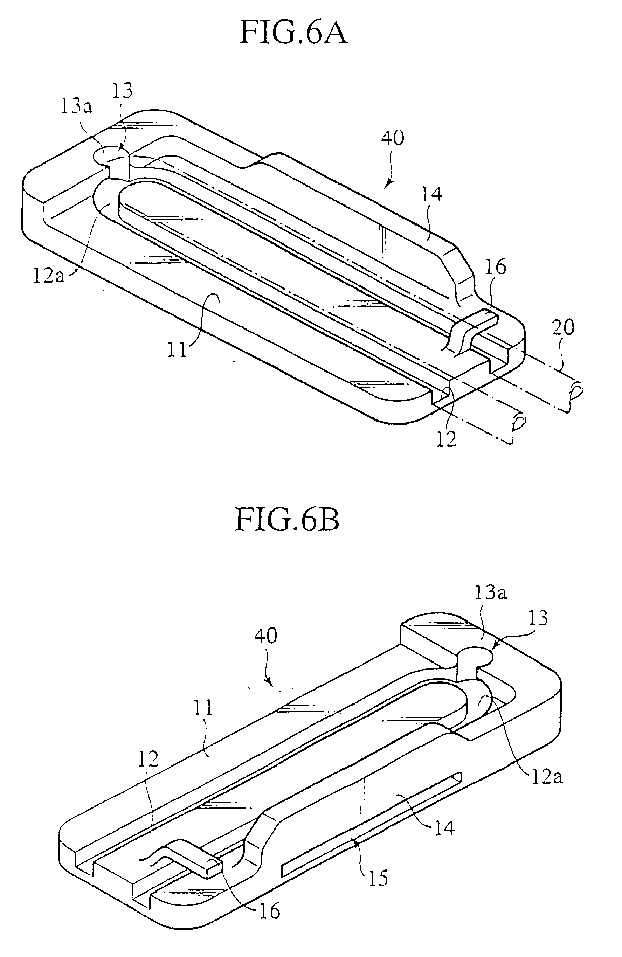

Figs. 6A, 6B. and 7 show a manufacturing jig for a medical treatment tube of another embodiment according to the invention.

In the following embodiments, substantially the same parts as those of the embodiments as shown on Figs. 1 to 5 are provided with the same characters, and their explanations are saved.

The manufacturing jig 40 as shown on Figs. 6A and 6B has a curved part 12a to bend and hold the tube 20 with a curvature without buckling. The curved part 12a has an arc shaped guide 13 formed on an outer circumference. That is, the guide part 13 has an arc shaped inner face a part of which is cut and removed by an outer circumference of the recessed part 12, By inserting the cylindrical blade 31 of the cutter 32 as shown on Fig. 1 along the guide part 13, a bent wall part on an outer circumference of the tube 20 inserted in the recessed part 12 is cut in an arc shape. At one end of the recessed part 12. a holding piece 16 for holding the inserted tube 20 is provided.

The base 11 has a rib 14 along a straight line shaped part of the recessed part 12 formed at one edge thereof. The rib 14 has a guide part 15 in a straight line formed at an outer face thereof, the guide part 15 opening to inside of the recessed part 12. The flat plate shaped blade 33 of the cutter 34 is inserted in the guide part 15 for cutting, and the tube 20 has a slit along a longitudinal direction formed at a circumferential wall thereof.

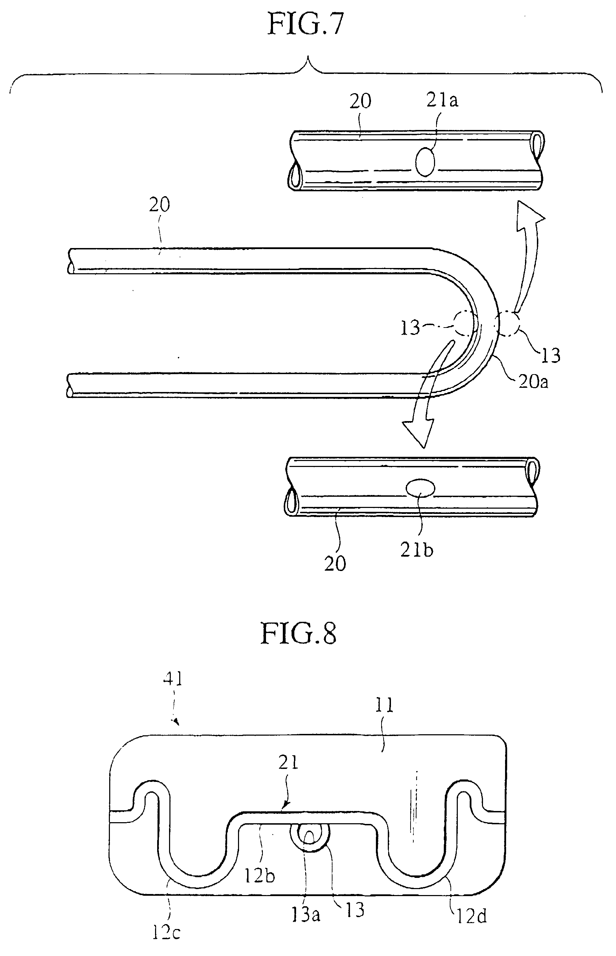

The guide part 13, as shown on Fig 7, may be provided on an inner circumference of a bent part 20a of the tube 20. If tube 20 has the guide part 13 on the outer circumference of the bent part 20a to be cut by the blade 31, when the tube 20 returns in the straight line shape, the tube 20 has a shortened hole 21a formed in a longitudinal direction thereof. If the tube 20 has the guide part 13 on an inner circumference of the bent part 20a thereof to be cut by the blade 31, when the tube 20 returns in the straight line shape, the tube 20 has an elongated hole 21b formed in the longitudinal direction thereof.

Fig. 8 shows a manufacturing jig for a medical treatment tube of further another embodiment according to the invention.

The manufacturing jig 41 has a recessed part 12 for holding a medical treatment tube on the base 11, the recessed part 12 having a shape in a straight line at a central part 12b in a longitudinal direction thereof, the central part 12b being provided with a cylindrical guide part 13, both of opposite side parts 12c, 12d relative to the guide part 13 having a shape in a zigzag line in the longitudinal direction. A straight line shaped guide part is saved.

In the manufacturing jig 41, when a tube for a medical treatment is inserted in the recessed part 12, the both of the side parts 12c, 12d are in a zigzag line, and holding forces of the parts are enhanced, thereby effectively preventing the tube from shift in the longitudinal direction and from rotation. Therefore, when in the guide part 13 provided at the central part 12b of the recessed part 12, the cylindrical blade is inserted for cutting, the tube does not move, and a hole in a perfect shape can be precisely formed at a predetermined position.

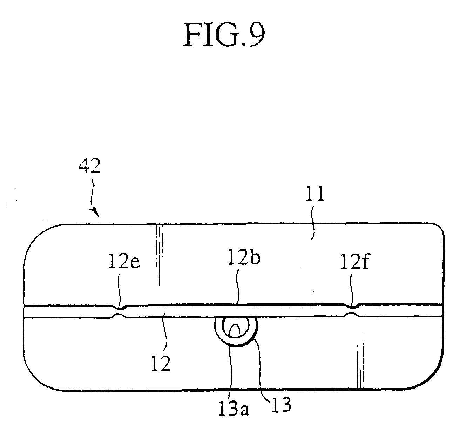

Fig. 9 shows a manufacturing jig for a medical treatment tube of furthermore another embodiment of the invention.

In the manufacturing jig 42 for a medical treatment tube, a recessed part 12 for holding a tube for a medical treatment provided on a base 11 is formed in a straight line as a whole, having a cylindrical guide part 13 provided at a central part in a longitudinal direction thereof. To both of opposite side parts relative to the guide part 13 in the longitudinal direction, inner walls of the recessed part 12 are projected inward to form width narrowed parts 12e, 12f. Similarity to the above embodiment, a guide part in a straight line are saved.

In the manufacturing jig 42, when the tube is inserted and held the width-narrowed parts 12e. 12f of the recessed parts 12 hold the tube especially tightly, thereby preventing the tube from shift in a longitudinal direction and from rotation. Therefore, when a cylindrical blade is inserted in the guide part 13 for cutting which is provided at the straight line shaped central part 12b of the recessed part 12, the tube does not move and a hole can be precisely formed in a perfect shape at a predetermined position.

As described above, due to securely holding the tube in the recessed part 12, the recessed part 12 may be in a zigzag line, a width-narrowed part may be provided, or an inner wall of the recessed part 12 may become a rough face to enhance a frictional force.

While preferred embodiments of the present invention have been described using specific terms, such description is for illustrative purposes, and it is to be understood that changes and variations may be made without departing from the spirit or scope of the following claims.

## Claims

1. A manufacturing jig for a medical treatment tube, comprising:
a recessed part configured to insert the tube to be held; and
a guide part configured to bring a cutter in contact with a side face of the tube to be held in the recessed part.

2. A manufacturing jig for a medical treatment tube according to claim 1.
wherein the cutter has a cylindrical or an arc shaped blade, and the guide part has a cylindrical or an arc shaped inner wall for guiding the blade, being formed such that a part of the blade contacts in an arc shape with a part of a circumferential wall of the tube in an arc shape in a crossing direction to a longitudinal direction of the tube when the cutter is inserted in the guide part.

3. A manufacturing jig for a medical treatment tube according to claim 1.
wherein the cutter has a blade in a straight line, and the guide part is formed in a straight line along a longitudinal direction of the tube.

4. A manufacturing jig for a medical treatment tube according to claims 1 to 3,
wherein the recessed part has a straight line shaped part for straightly holding the tube, and the guide part is provided to the straight line shaped part.

5. A manufacturing jig for a medical treatment tube according to claim 2,
wherein the recessed part has a curved part with a curvature for bending and holding the tube without buckling, the curved part has the guide part provided on an inner or an outer circumference thereof.

6. A manufacturing jig for a medical treatment tube according to claims 1 to 5,
wherein the recessed part has a width smaller than a diameter of the tube and capable of inserting the tube under pressure, and, has a depth for a part of a circumferential wall of the tube to project from an opening edge thereof when inserting the tube under pressure.

7. A manufacturing jig for a medical treatment tube according to claims 1 to 6,
wherein width narrowed parts are provided at opposite positions relative to a part of the recessed pare provided with a guide part, for the tube to be held further tightly.

8. A manufacturing jig for a medical treatment tube according to claim 1,
wherein the recessed part has a wall, and the guide part opens the wall.

9. A manufacturing jig for a medical treatment tube according to claim 8,
wherein the wall straightly extends.

10. A manufacturing jig for a medical treatment tube according to claim 8,
wherein the wall is curved.

11. A manufacturing jig for a medical treatment tube according to claim 9,
wherein the guide part extends along the wall.

12. A manufacturing jig for a medical treatment tube according to claim 8,
wherein the guide part opens an end of the wall.

13. A manufacturing jig for a medical treatment tube according to claim 8.
wherein the recessed part is narrowed at a place thereof.

14. A device (10;40;41;42) for guiding cutting means (31, 32; 33, 34) relative to a medical treatment tube (20), the device comprising tube holding means (12; 12, 16; 12b, 12c, 12d; 12, 12b, 12e, 12f) for holding the medical treatment tube such that the cutting means are guided by guide means (13, 13a, 15, 15a, 15b) to cut the medical treatment tube.
